(19) Europäisches Patentamt
European Patent Office
Office européen des brevets

(11) Publication number: **0 043 194**
**A2**

(12) # EUROPEAN PATENT APPLICATION

(21) Application number: 81302546.7

(22) Date of filing: 09.06.81

(51) Int. Cl.³: C 07 C 87/457
//C07C103/737, C07C121/75,
C07C61/39

(30) Priority: 19.06.80 GB 8020129

(43) Date of publication of application:
06.01.82 Bulletin 82/1

(84) Designated Contracting States:
AT BE CH DE FR GB IT LI LU NL SE

(71) Applicant: FISONS LIMITED
Fison House 9 Grosvenor Street
London WIX OAH(GB)

(72) Inventor: Brown, Kevan
329 Beacon Road
Loughborough Leicestershire(GB)

(74) Representative: Craig, Christopher Bradberry et al,
Fisons Limited Fison House Princes Street
Ipswich 1P1 1QH(GB)

(54) **Bicyclo-octatriene derivatives, pharmaceutical compositions containing them and processes and intermediates for their production.**

(57) There are described compounds of formula I

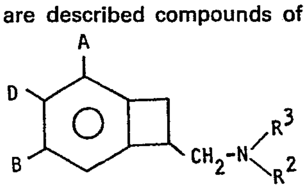

1

in which one of A, B and D represents hydrogen and the other two independently represent $-CH_2OR$ or -OR,

R represents hydrogen or phenylalkyl, and

$R^2$ and $R^3$, which may be the same or different, each represent hydrogen, alkyl or unsubstituted or substituted phenylalkyl,

and the pharmaceutically acceptable salts thereof.

There are also described processes for producing the compounds by reduction or hydrolysis and pharmaceutical, e.g. cardiac, compositions containing them.

compound of formula II,

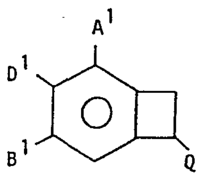

11

in which one of $A^1$, $B^1$ and $D^1$ represents hydrogen and the other two independently represent -CHO, $-CH_2OR^1$ or $-OR^1$,

$R^1$ represents a blocking group or a group R as defined above, and

Q represents $-CH_2NR^2R^3$, -CN or $-CONR^2R^3$ where $R^2$ and $R^3$ are as defined above, provided that $R^1$ does not represent hydrogen when Q represents $-CH_2NR^2R^3$,

EP 0 043 194 A2

0043194

4975/111



BA 80/20129

BICYCLO-OCTATRIENE DERIVATIVES, PHARMACEUTICAL COMPOSITIONS CONTAINING THEM AND PROCESSES AND INTERMEDIATES FOR THEIR PRODUCTION

This invention concerns certain pharmaceutically-active compounds, processes for their preparation, and compositions containing them.

Certain cyclobutene derivatives are described, e.g. in British Patent Specification No. 1,042,197, as being of use as intermediates for use in the production of medicaments and also as having analgesic properties. We have now found that a particular group of aminomethyl bicyclo octatrienes possess unexpected properties in that they can influence the functioning of the cardiovascular system.

In one aspect, this invention provides the bicyclo-[4.2.0]octa-1,3,5-triene-7-methanamines of formula I,

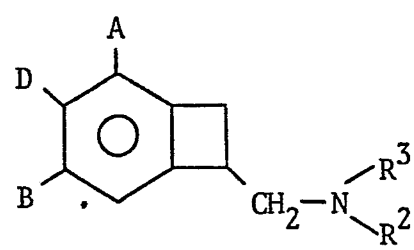

I

in which one of A, B and D represents hydrogen and the other two independently represent $-CH_2OR$ or -OR,

R represents hydrogen or phenylalkyl, and

$R^2$ and $R^3$, which may be the same or different, each represent hydrogen, alkyl or unsubstituted or substituted phenylalkyl,

and the pharmaceutically acceptable salts thereof.

In another aspect, this invention provides a process

for the preparation of a compound of formula I, or a pharmaceutically acceptable salt thereof, wherein a bicyclo[4.2.0]octa-1,3,5-triene of formula II,

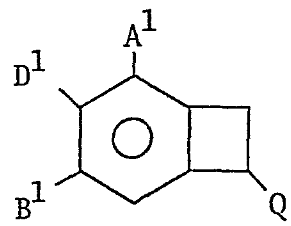

II

in which one of $A^1$, $B^1$ and $D^1$ represents hydrogen and the other two independently represent -CHO, $-CH_2OR^1$ or $-OR^1$,

$R^1$ represents a blocking group or a group R as defined above, and

Q represents $-CH_2NR^2R^3$, -CN or $-CONR^2R^3$ where $R^2$ and $R^3$ are as defined above, provided that $R^1$ does not represent hydrogen when Q represents $-CH_2NR^2R^3$,

is reduced or hydrolysed to give the corresponding compound of formula I,

and where desired or necessary converting the resulting compound of formula I to a pharmaceutically acceptable salt thereof or vice versa.

When Q represents -CN, the resulting compound of formula I is one in which $R^2$ and $R^3$ both represent hydrogen. The reduction of the group Q may be effected by any appropriate means, for example by means of borane, lithium aluminium hydride, sulphuretted sodium borohydride or catalytically using hydrogen, e.g. in the presence of a

palladium catalyst. Alternatively, where Q represents a group $-CONR^2R^3$, aluminium hydride may be employed. The non-catalytic reduction of a group Q is preferably carried out in a solvent which is inert under the reaction conditions, e.g. diethyl ether, glyme, dioxan or tetrahydrofuran. The reduction preferably takes place at a temperature of from $0^o$ to $100^oC$ and also preferably in the presence of excess reducing agent. The reduction of the groups $-CH_2OR^1$ or $-OR^1$ where $R^1$ represents a blocking group, e.g. benzyl, may be effected by any suitable method, e.g. catalytically using hydrogen (e.g. at 1 to 4 atmospheres pressure) over a palladium/charcoal catalyst and in the presence of a solvent which is inert under the reaction conditions e.g. ethanol or acetic acid. The reduction of a group Q and $A^1$, $B^1$ or $D^1$, may, if desired by carried out simultaneously. When a blocking group or a group R is removed from a compound of formula II the process may be reduction or hydrolysis. The hydrolysis may be carried out using an acid, e.g. HCl, HBr or HI and at a temperature of from 0 to $200^o$, preferably 10 to $100^oC$. Alternatively the hydrolysis may be effected by means of a boron trihalide, e.g. boron tribromide or boron trichloride, at a temperature of from -80 to $+100^oC$ and

preferably in the presence of a chlorocarbon solvent, e.g. dichloroethane, chloroform or dichloromethane.

The compounds of formula II, some of which are new compounds, e.g. when $R^1$ represents benzyl, may be prepared, where Q represents a group $-CONR^2R^3$, by a process in which a bicyclo[4.2.0]octa-1,3,5-triene of the formula III,

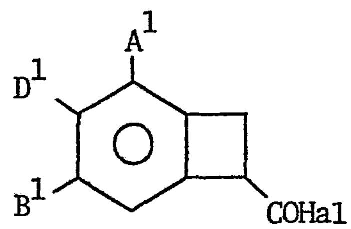

III

in which $A^1$, $B^1$ and $D^1$ are as defined above, and Hal represents halogen, e.g. chlorine or bromine,

is reacted with an amine of the formula $R^2R^3NH$.

The compounds of formula III, some of which are new compounds, e.g. when $R^1$ represents benzyl, may be prepared by a process in which a bicyclo[4.2.0]octa-1,3,5-triene-7-carboxylic acid of the formula IV,

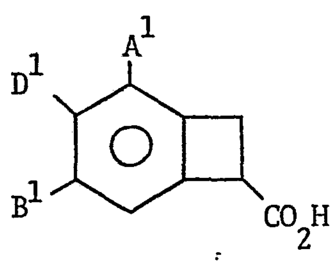

IV

in which $A^1$, $B^1$ and $D^1$ are as defined above, is halogenated by means of a suitable halogenating agent.

The halogenating agent may be, for example, a thionyl

halide $SOHal_2$, a phosphorus halide $PHal_5$, or a phosphoryl halide $POHal_3$. The reaction is conveniently effected in an appropriate solvent medium, e.g. toluene or 1,2-dichloroethane.

The bicyclo[4.2.0]octa-1,3,5-triene-7-carboxamides of formula II may alternatively be prepared in one stage from the compounds of formula IV by reaction with dicyclohexylcarbodiimide, or a suitable dehydrating/coupling reagent, and the appropriate amine $R^2R^3NH$ in which $R^2$ and $R^3$ are as defined above.

The 7-carboxylic acids of formula IV, some of which are new compounds, may be prepared by hyrolysis of the corresponding compound of formula II in which Q is -CN.

The hydrolysis is conveniently effected by means of an alkali-metal base, e.g. sodium or potassium hydroxide, suitably in an appropriate solvent medium, e.g. ethanol.

The compounds of formula II in which Q is -CN, some of which are new compounds, may be prepared by a process in which a compound of the formula V,

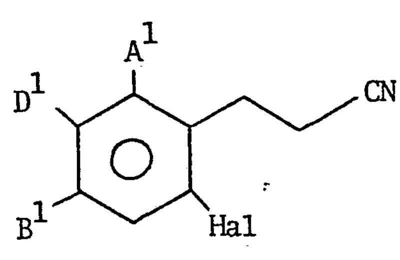

V

in which $A^1$, $B^1$, $D^1$ and Hal are as defined above, is subjected to the action of a strong base to give the

desired compound.

The strong base is conveniently an alkali-metal amide or alkoxide or an alkyl alkali-metal e.g. butyl lithium, sodium amide, lithium diethylamide, lithium di-isopropylamide or potassium-t-butoxide.

The compounds of formula V are either known or may be made from known compounds using conventional processes known *per se*.

In the production of above intermediates it may be necessary or desirable to protect any formyl group which is present during one or more of the steps.

Compounds of formula II in which one of the substituents is a formyl group may also be made by the process disclosed in Example 7 or processes analagous thereto.

Once prepared the compounds of formula I may be converted into other compounds of formula I, especially where different groups $R^2$ and/or $R^3$ are desired, by methods known *per se*.

Acid addition salts may be prepared by reacting the free compound of formula I with the appropriate acid. The free compounds of formula I may be regenerated from the acid addition salts by reaction with an appropriate base.

This invention naturally extends to the compounds of formula I and the acid addition salts thereof whenever prepared by a process as described.

The salts of the compounds of formula I are preferably those formed with a mineral or organic acid having a physiologically-acceptable anion, e.g. a hydrohalic acid such as hydrochloric, hydrobromic or hydriodic acid, citric acid or tartaric acid.

The compounds of the invention are useful because they possess pharmacological activity in animals, in particular they act on peripheral and/or central dopaminergic receptors. As such, they lower blood pressure, reduce heart rate and increase blood flow to contain vascular beds, e.g. the kidneys. The compounds also have an action on other adrenoreceptors, and these exhibit a positive inotropic effect. Activity of the compounds has been observed in the following assay systems:

(a) canine renal blood flow, McNay and Goldberg, J. Pharmac, Exp. Ther., 151, 23-31, 1966.

(b) rabbit isolated ear artery, McCullogh, Rand and Story, Br. J. Pharmac, 49, 141-142, 1973, and

(c) cat nictitating membrane, Gyorgy and Doda, Arch, Int. Pharmacodyn, 226, 194-206, 1977.

The compounds of the invention are indicated for use in the treatment of congestive heart failure, renal failure, angina pectoris, ischaemic heart disease and hypertension, hyperprolactinaemia and also in Parkinson's disease, and other neurological disorders.

The dosage administered will naturally depend on the compound employed, the mode of administration and the desired effect. However, in general, satisfactory results are obtained when the compounds are administered at a dosage of from 0.05µg to 10mg per kilogram of body weight per day. For man, the indicated total daily dosage is in the range 2.5µg to 300mg, which may be administered in divided doses for example 2 to 6 times a day, or in sustained release form. Thus unit dose forms may comprise from about 1µg to 200mg of the compound.

The compounds of formula I and their pharmaceutically acceptable salts are advantageous in that they possess a different spectrum of activities, e.g. they possess less undesirable side effects, have advantageous absoption profile, are more potent, are more active and possess other desirable properties as compared to structurally similar compounds.

We prefer A or B to be hydrogen and the remaining pair of A, B and D to be -OR. R preferably represents hydrogen. $R^2$ and/or $R^3$ are preferably hydrogen or alkyl C 1 to 6, especially C 1 to 4, for example methyl, ethyl, n-propyl, isopropyl or t-butyl. When R, $R^2$ and/or $R^3$ represents phenylalkyl, the alkyl moiety is preferably C 1 to 4 and especially C 1 or 2, e.g. benzyl. Where the phenylalkyl group is substituted, the substituent group(s)

are preferably alkyl or alkoxy, preferably C 1 to 4, e.g. methyl, ethyl, methoxy or ethoxy; halogen, e.g. chlorine or bromine, or nitro groups, e.g. 3,4-dimethoxyphenylethyl.

Specifically preferred compounds of formula I are those of Examples 5 and 6, and the pharmaceutically acceptable salts thereof.

According to our invention we also provide a pharmaceutical composition comprising (preferably less than 80%, and more preferably less than 50% by weight) of a compound of formula I, or a pharmaceutically acceptable salt thereof, in admixture with a pharmaceutically acceptable adjuvant, diluent or carrier. Examples of suitable adjuvants, diluents or carriers are:- for tablets, capsules and dragées; microcrystalline cellulose, calcium phosphate, diatomaceous earth, a sugar such as lactose, dextrose or mannitol, talc, stearic acid, starch, sodium bicarbonate and/or gelatin; for suppositories, natural or hardened oils or waxes; and for inhalation compositions, coarse lactose. The compound of formula I, or the pharmaceutically acceptable salt thereof, preferably is in a form having a mass median diameter of from 0.01 to 10 microns. The compositions may also contain suitable preserving, stabilising and wetting agents, solubilizers, sweetening and colouring agents and flavourings. The compositions may, if desired, be formulated in sustained release form.

We prefer compositions which are designed to be taken oesophageally and to release their contents in the gastrointestinal tract.

The compounds of formula I are asymetric and may therefore exist in the form of two optical isomers or a racemic or other mixture of such isomers. The various optical isomers may be resolved, wholly or partially, using conventional techniques, e.g. formation of a salt with an optically active acid, fractional crystallisation of the salt and subsequent regeneration of the free base.

In another aspect the invention provides a method of increasing the force of contraction of the heart in an animal, either human or non-human, which method comprises administering to the animal an effective amount of one or more compounds of the invention.

The following Examples are now given, though only by way of illustration of the invention. All temperatures quoted are in $^{o}C$.

Example 1

3,4-Dihydroxybicyclo[4.2.0]octa-1,3,5-triene-7-methanamine, hydrochloride

(a) 3,4-Dihydroxybicyclo[4.2.0]octa-1,3,5-triene-7-carbonitrile

A solution of 3,4-dimethoxybicyclo[4.2.0]octa-1,3,5-triene-7-carbonitrile (5.0gm, 0.0265M) in dry 1,2-dichloroethane (150ml) was stirred in an atmosphere of nitrogen at -40$^{o}$C while boron tribromide (7.5ml, 0.08M) was added dropwise. The mixture was allowed to warm to room temperature and then was stirred overnight. Water (100ml) was added cauuiously and the organic layer was separated. The aqueous layer was washed with chloroform and the combined organic layers were washed with water, dried over magnesium sulphate, filtered and evaporated to dryness leaving a colourless oil (3.0gm, 70%) which slowly solidified.

(b) <u>3,4-Dihydroxybicyclo[4.2.0]octa-1,3,5-triene-7-methanamine, hydrochloride</u>

A solution of 3,4-dihydroxybicyclo[4.2.0]octa-1,3,5-triene-7-carbonitrile (3.9gm, 0.0242M) in dry tetrahydrofuran (50ml) was stirred at room temperature under nitrogen while borane/tetrahydrofuran complex (90ml, 0.09M) was added dropwise. The mixture was stirred at room temperature under nitrogen for 5 hours. Ethanol (20ml) was added cautiously followed by saturated ethanolic hydrogen chloride (20ml) then stirring was continued overnight at room temperature. Dry diethyl ether was added in excess and the solid obtained was filtered, washed with hot ethanol and dried to give 3,4-dihydroxybicyclo[4.2.0]-octa-1,3,5-triene-7-methanamine hydrochloride as a white powder, .6gm (33%) mp 208$^{o}$C.

Analysis

| | Found: | C 52.4; | H 6.0; | N 6.9% |
|---|---|---|---|---|
| $C_9H_{11}NO_2$.HCl requires: | | C 52.4; | H 6.0; | N 6.8% |
| (with 0.25 $H_2O$) | | | | |

Example 2

3,4-Dihydroxybicyclo[4.2.0]octa-1,3,5-triene-7-N[2-(3,4-dimethoxyphenyl)ethyl]methanamine, hydrochloride

(a) 3-(2-Bromo-4,5-dibenzyloxyphenyl)propionitrile

To a solution of 3-(3,4-dibenzyloxyphenyl)propionitrile (23.7g; 0.069M) in carbon tetrachloride (165ml) was added N-Bromosuccinimide (16.0g; 0.09M) and the mixture was heated at reflux for 3 hours. The solution was cooled, washed with water, IN aq sodium hydroxide solution and water, and was dried over magnesium sulphate. The organic solution was evaporated to dryness to leave an oil which slowly solidified on cooling. The solid was crystallised twice from methanol to give the bromo-derivative 8.1g (27%) mp 81-3$^{o}$.

Analysis

| | Found: | C 65.8; | H 4.7; | N 3.0% |
|---|---|---|---|---|
| $C_{23}H_{20}BrNO_2$ requires: | | C 65.4; | H 4.7; | N 3.3% |

(b) 3,4-Dibenzyloxybicyclo[4.2.0]octa-1,3,5-triene-7-carbonitrile

A solution of butyl lithium (2.5M in hexane; 66ml, 0.165M) was added from a syringe, under nitrogen to dry

tetrahydrofuran cooled at $-20^{\circ}$ (250ml). The resulting solution was stirred while dry diethylamine was added (17.3ml; 0.165M). This mixture was stirred for 10 minutes at $-20^{\circ}$. A solution of 3-(2-bromo-4,5-dibenzyloxyphenyl)propionitrile (21.1g; 0.05M) in dry tetrahydrofuran (50ml) was added dropwise with stirring under nitrogen, ensuring that the temperature did not rise above $-15^{\circ}$. Stirring was continued for 3 hours and then the mixture was quenched by addition of 10% aqueous hydrochloric acid (400ml). The aqueous phase was extracted with ethyl acetate and the combined organic layers were washed well with water and dried over magnesium sulphate. The solution was evaporated to dryness leaving an oil, 16.2g (95%).

(c) <u>3,4-Dibenzyloxybicyclo[4.2.0]octa-1,3,5-triene-7-carboxylic acid</u>

3,4-Dibenzyloxybicyclo[4.2.0]octa-1,3,5-triene-7-carbonitrile (12.0g; 0.035M) was suspended in saturated ethanolic potassium hydroxide (30ml) overnight. Water (10ml) was then added and the mixture was heated at reflux for 1 hour then poured into water (250ml). The aqueous solution was washed well with diethyl ether and then acidified to pH 1 with dilute hydrochloric acid. The oil which precipitated was extracted into diethyl ether and this solution was dried over magnesium sulphate, filtered and the filtrate was evaporated to dryness leaving a

sticky solid. This was triturated with petroleum ether (40-60°) to give a fawn solid 7.0g (55%) mp 100-102°.

(d) 3,4-Dibenzyloxybicyclo[4.2.0]octa-1,3,5-triene-7-N-[2-(3,4-dimethoxyphenyl)ethyl]carboxamide

3,4-Dibenzyloxybicyclo[4.2.0]octa-1,3,5-triene-7-carboxylic acid (29.0g; 0.08M) and thionyl chloride (12.08g; 0.101M) were heated at reflux in dry toluene (500ml) for 1 hour. The solution was evaporated to dryness leaving a red oil.

This oil was stirred in toluene (200ml) while a solution of homoveratrylamine (34.5g; 0.19M) in toluene (50ml) was quickly added, with cooling. The mixture was then stirred for 30 minutes, filtered and the solid was taken up in chloroform. This solution was washed with dilute hydrochloric acid and dried over magnesium sulphate, filtered and evaporated to dryness to leave a solid which was crystallised from ethanol to give the amide, 30.0g (71%) mp 156°.

Analysis

| | | | |
|---|---|---|---|
| Found: | C 75.4; | H 6.5; | N 2.8% |
| $C_{33}H_{33}NO_5$ requires: | C 75.7; | H 6.3; | N 2.7% |

(e) 3,4-Dibenzyloxybicyclo[4.2.0]octa-1,3,5-triene-7-N-[2-(3,4-dimethoxyphenyl)ethyl]methanamine, hydrochloride

A suspension of 3,4-dibenzyloxybicyclo[4.2.0]octa-1,3,5-triene-7-N-[2-(3,4-dimethoxyphenyl)ethyl]carboxamide

(10.4g; 0.02M) in dry tetrahydrofuran (500ml) was stirred while borane/tetrahydrofuran complex (100ml, 0.1M) was added under nitrogen. The resulting solution was heated under nitrogen for 3 hours, cooled and ethanol (20ml) was cautiously added followed 15 minutes later by ethanolic hydrogen chloride (25ml). The solution was stirred for 1 hour, evaporated to dryness and the residue was washed well with dry diethyl ether. The solid was stirred at room temperature with ethanol (100ml) overnight, filtered and dried to give the required compound 10.1g (93%) mp 112-3°C.

<u>Analysis</u>

| | | | |
|---|---|---|---|
| Found: | C 72.7; | H 6.6; | N 2.6% |
| $C_{33}H_{35}NO_4$.HCl requires: | C 72.59; | H 6.59; | N 2.56% |

(f) <u>3,4-Dihydroxybicyclo[4.2.0]octa-1,3,5-triene-7-N-[2-(3,4-dimethoxyphenyl)ethyl]methanamine, hydrochloride</u>

A solution of 3,4-dibenzyloxybicyclo[4.2.0]octa-1,3,5-triene-7-N[2-(3,4-dimethoxyphenyl)ethyl]methanamine hydrochloride (4.0g, 0.0074M) in ethanol (200ml) containing concentrated hydrochloric acid (10 drops) was hydrogenated at 3 atmospheres pressure and 30°C over 5% Pd/C (0.4g). After this time a further amount of catalyst was added and hydrogenation was continued. The catalyst was removed by filtration and the filtrate was evaporated to dryness. The residue was crystallised from ethanol to give the

required compound 2.0g (74%) mp 208° (d).

Analysis

Found: C 58.8; H 6.3; N 3.8%

$C_{19}H_{23}NO_4$.HCl requires with 0.61M excess HCl

C 58.8; H 6.3; N 3.6%

Example 3

3,4-Dihydroxybicyclo[4.2.0]octa-1,3,5-triene-7-N-(1-methylethyl]methanamine, hydrochloride

(a) 3,4-Dibenzyloxybicyclo[4.2.0]octa-1,3,5-triene-7-N-[1-methylethyl]carboxamide

A mixture of 3,4-dibenzyloxybicyclo[4.2.0]octa-1,3,5-triene-7-carboxylic acid (6.0g, 0.0167M), thionyl chloride (2.5g, 0.02M) and toluene (250ml) was heated at reflux for 1 hour. The reaction mixture was then evaporated under reduced pressure leaving a dark brown oil. To a solution of this oil in toluene (100ml) was added isopropylamine (2.4g, 0.04M) and the mixture was stirred. The solid which precipitated was filtered off, dissolved in ethyl acetate and this solution was washed with water and dilute hydrochloric acid. It was dried over magnesium sulphate, filtered and the filtrate was evaporated to dryness leaving the required compound as a fawn solid, 4.0g (60%) mp 137-9°C.

Analysis

Found: C 77.4; H 7.1; N 3.2%

$C_{26}H_{27}NO_3$ requires: C 77.8; H 6.8; N 3.5%

(b) 3,4-Dibenzyloxybicyclo[4.2.0]octa-1,3,5-triene-7-N-[1-methylethyl]methanamine, hydrochloride

A solution of 3,4-dibenzyloxybicyclo[4.2.0]octa-1,3,5-triene-7-N-[methylethyl]carboxamide (11.0g, 0.027M) was stirred under nitrogen in dry tetrahydrofuran (175ml) while a solution of borane-tetrahydrofuran complex (137ml, 0.137M) was added. The resulting solution was heated and stirred at reflux for 4 hours. Ethanol (350ml) was added cautiously followed by ethanolic hydrogen chloride (50ml) and the resulting solution was evaporated to small volume and cooled. A white solid was obtained which was crystallised from propan-2-ol to give the required compound 6.5g (56%) mp 178-9°.

Analysis

| | | |
|---|---|---|
| | Found: | C 73.5; H 7.2; N 3.0; Cl 8.7% |
| $C_{26}H_{29}NO_2$.HCl requires | | C 73.6; H 7.3; N 3.3; Cl 8.4% |

(c) 3,4-Dihydroxybicyclo[4.2.0]octa-1,3,5-triene-7-N-[1-methylethyl]methanamine, hydrochloride

A solution of 3,4-dibenzyloxybicyclo[4.2.0]octa-1,3,5-triene-7-N-[1-methylethyl]methanamine, hydrochloride (6.0g, 0.014M) in ethanol (450ml) containing concentrated hydrochloric acid (20 drops) was hydrogenated at 30 psi over 5% palladium/charcoal (0.9g) for 45 minutes. The catalyst was removed by filtration and the filtrate was evaporated to dryness. The sticky residue was triturated

with propan-2-ol to give an off-white solid 2.75g (80%) mp 178-180°.

Analysis

| | | |
|---|---|---|
| | Found: | C 59.0; H 7.5; N 5.7; Cl 14.6% |
| $C_{12}H_{17}NO_2$.HCl requires: | | C 59.1; H 7.4; N 5.75; Cl 14.5% |

Example 4

2,3-Dihydroxybicyclo[4.2.0]octa-1,3,5-triene-7-(N,N-dimethyl)-methanamine hydrobromide

(a) 2,3-Dimethoxybicyclo[4.2.0]octa-1,3,5-triene-7-carboxylic acid

A mixture of 2,3-dimethoxybicyclo[4.2.0]octa-1,3,5-triene-7-carbonirile (15.0g, 0.079M) and saturated ethanolic potassium hydroxide (100ml) was stirred at room temperature for 24 hours. Water (20ml) was added and the solution was heated at reflux for 3 hours, then poured into a large excess of cold water and this aqueous solution was washed with diethyl ether. The solution was acidified with concentrated hydrochloric acid and extracted with chloroform. The extracts were washed well with water, dried over magnesium sulphate, filtered and evaporated to dryness leaving a fawn solid, 15.0g (91%) mp 120°.

(b) 2,3-Dimethoxybicyclo[4.2.0]octa-1,3,5-triene-7-(N,N-dimethyl)carboxamide

A mixture of 2,3-dimethoxybicyclo[4.2.0]octa-1,3,5-triene-7-carboxylic acid (15.0g, 0.072M) and thionyl

chloride (18.04g, 0.15M) was heated at 100°C for 1 hour in toluene (150ml). The solution was evaporated to dryness and the residual oil was dissolved in toluene (100ml) to which was added 33% ethanolic dimethyl amine solution (20g, 0.145M). The resulting mixture was heated on a steam bath for 2 hours. The solution was evaporated to dryness and the product was chromatographed on a silica column eluting first with toluene/ethyl acetate (3:1) to remove unwanted ester and then with toluene/methanol (1:1) to remove the required amide which was isolated as a white solid 7.3g (43%) mp 90-91°.

(c) <u>2,3-Dimethoxybicyclo[4.2.0]octa-1,3,5-triene-7-(N,N-dimethyl)methanamine, hydrochloride</u>

A solution of 2,3-dimethoxybicyclo[4.2.0]octa-1,3,5-triene-7-(N,N-dimethyl)carboxamide (12.0g, 0.05M) in dry tetrahydrofuran (300ml) was stirred under nitrogen while a solution of borane-tetrahydrofuran complex was added (100ml, 0.1M). The mixture was stirred for 4 hours at room temperature, then ethanol (25ml) was added cautiously followed by ethanolic hydrogen chloride (25ml). The resulting mixture was stirred overnight. The white solid was removed by filtration, and was crystallised from propan-2-ol to give the required compound 1.7g (13%) mp 209-210°C.

(e) 2,3-Dihydroxybicyclo[4.2.0]octa-1,3,5-triene-7-(N,N-dimethyl)methanamine hydrobromide

To a suspension of 2,3-dimethoxybicyclo[4.2.0]octa-1,3,5-triene-7-(N,N-dimethyl)methanamine hydrochloride (2.3g) in dry dichloromethane (40ml) in an atmosphere of nitrogen, and cooled to $0^o$, was added a solution of boron tribormide (19.3ml) in dichloromethane, with vigorous stirring. The mixture was allowed to warm to room temperature and was stirred for 2 hours. Methanol (50ml) was cautiously added and the mixture was evaporated to dryness. The residue was dried by azeotropic distillation, washed well with hot ethyl acetate and crystallised from propan-2-ol to give the title compound as a white solid, 1.0g (49%) mp 142-$6^o$(d).

Analysis

Found: C 47.13; H 6.02; N 4.83%

$C_{11}H_{15}NO_2$.HBr requires with 2.02% water:

C 47.13; H 5.95; N 5.0%

Example 5

2,3-Dihydroxybicyclo[4.2.0]octa-1,3,5-triene-7-(N,N-di-n-propyl)methanamine hydrochloride

By methods analogous to those of Examples 1-4 the above compound was prepared via the corresponding 2,3-dibenzyloxy compounds, namely the 7-carboxylic acid, the 7-(N,N-di-n-propyl)carboxamide and 2,3-dibenzyloxy-

bicyclo[4.2.0]octa-1,3,5-triene-7-(N,N-di-n-propyl)methanamine (m.p. 148°C). The melting point of the product was 158-159°C.

Analysis

Found: C 60.09; H 8.05; N 4.69; Cl 12.12%

$C_{15}H_{23}NO_2$.HCl requires with 4.5% water

C 60.2; H 8.5; N 4.68; Cl 11.87%

Example 6

3,4-Dihydroxybicyclo[4.2.0]octa-1,3,5-triene-7-(N,N-di-n-propyl)methanamine hydrochloride

(a) 2-Cyano-3',4'-dibenzyloxycinnamic acid

A mixture of 3,4-dibenzyloxybenzaldehyde (120g, 0.377 mole), cyanoacetic acid (30g, 0.353 mole), pyridine (80ml) and ammonium acetate (5g) in toluene (600ml) was heated to reflux using a Dean and Stark water separator. After 4 hours reflux the reaction mixture was cooled and the solid isolated by filtration and recrystallised from ethanol affording the sub-title product as yellow needles (112g, 77%) mp 190-2°C.

(b) 2-Cyano-3-(3,4-dibenzyloxyphenyl)propionic acid

The product of step (a) (30g, 0.078 mole) was stirred in warm ethanol (1500ml), saturated sodium bicarbonate solution (200ml) and water (500ml) added and the mixture stirred until the yellow colour had faded. To the clear solution was added sodium borohydride (20g) in small

portions and the mixture stirred at 35°C for 1 hour. The majority of the ethanol was removed in vacuo and the aqueous residue adjusted to pH 5 with concentrated hydrochloric acid and stirred for 18 hours at room temperature. The white solid was isolated by filration and dried affording the sub-title product (30g, 100%) mp 94-7°C.

(c) 3-(3,4-Dibenzyloxyphenyl)propionitrile

The product of step (b) (110g, 0.284 mole) was heated to reflux in dimethylacetamide (500ml) for 2½ hours. The mixture was cooled and poured into a well stirred mixture of ice and water (3000ml). Stirring was continued for 2 hours, and the resulting solid isolated by filtration and air dried affording the sub-title product (91.5g, 94%) mp 48-50°C.

(d) 3-(6-Bromo-3,4-dibenzyloxyphenyl)propionitrile

The product of step (c) (87g, 0.253 mole) and N-bromosuccinimide (52.gg, 0.29 mole) in carbontetrachloride (600ml) were heated to reflux with stirring for 2 hours. Water was then added and the organic layer washed with water, IN aqueous sodium hydroxide solution, water again, dried ($MgSO_4$) filtered and evaporated affording the crude product as a yellow oil. This oil was crystallised and recrystallised from methanol affording the sub-title product (78.5g, 73%) mp 82-3°C.

(e) 3,4-Dibenzyloxybicyclo[4.2.0]octa-1,3,5-triene-7-carboxylic acid

Diethylamine (17.5ml, 0.065 mole) was added to a stirred solution of n-butyllithium (146ml of a 1.15M solution in hexane, 0.168 mole) in dry tetrahydrofuran (350ml) at -15°C under dry nitrogen. After stirring for 15 minutes a solution of the product of step (d) (21.0g, 0.05 mole) in dry tetrahydrofuran (100ml) was added over a period of 20 minutes at -15°C and stirring continued for a further 2½ hours. Dilute HCl was added to quench the reaction and the mixture extracted with chloroform. The combined chloroform extracts were washed with dilute HCl, water and saturated aqueous sodium bicarbonate solution, dried ($MgSO_4$), filtered and evaporated affording a red-brown oil (15.0g). This oil was chromatographed on silica-gel eluting with toluene and then toluene-ethylacetate (9:1) affording the crude nitrile (11.1g) which was hydrolysed without further purification.

The crude nitrile was heated to reflux for 1 hour with a mixture of saturated ethanolic potassium hydroxide (100ml) and water (50ml). The cooled reaction mixture was poured into water and washed well with ether. The aqueous solution was acidified with concentrated hydrochloric acid, cooled in ice, and stirred vigorously for 1 hour. The pale brown solid was isolated by filtration and dried

in air affording the sub-title product (8.7g, 74%) mp 103-5°C.

n.m.r. (DMSO-$d_6$) δ: 3.15(2H,d); 4.1(1H,t); 5.1(4H,s); 6.9(2H,s); 7.4(10H,m).

(f) 3,4-Dibenzyloxybicyclo[4.2.0]octa-1,3,5-triene-7-(N,N-di-n-propyl)carboxamide

A mixture of the product of step (e) (0.72g, 0.002 mole), thionylchloride (0.2ml, 0.0027 mole) and dimethylformamide (1 drop) in toluene (20ml) was heated to reflux for 1 hour. The solution was evaporated to dryness and several portions of toluene added and again evaporated to dryness. The residue was dissolved in dry toluene, and di-n-propylamine (0.4g, 0.004 mole) added. The mixture was then stirred for 1 hour at room temperature, washed with dilute HCl, water and saturated sodium bicarbonate solution, dried ($MgSO_4$) filtered and evaporated affording the sub-title product as a brown oil (0.7g, 79%).

n.m.r. ($CDCl_3$) δ: 0.95(6H,m); 1.6(4H,m); 3.3(6H,m); 4.3(1H,t); 5.1(4H,s); 6.8(2H,s); 7.4(10H,m).

(g) 3,4-Dihydroxybicyclo[4.2.0]octa-1,3,5-triene-7-(N,N-di-n-propyl)methanamine

The product of step (f) (16.6g, 0.037 mole) was heated to reflux in dry tetrahydrofuran (200ml) with diborane (160ml of a 1M solution in tetrahydrofuran, 0.16 mole) for 5 hours. The cooled reaction mixture was stirred at room

temperature for 18 hours, the excess diborane destroyed with methanol and the reaction mixture evaporated to dryness. Methanol and a solution of HCl gas in ether were added to the residue and the mixture heated to reflux for 2 hours and evaporated to dryness affording the crude dibenzyloxy amine hydrochloride. This intermediate was hydrogenated at atmospheric pressure with 5% palladium on carbon (1.4g) in ethanol (200ml) for 4½ hours. The catalyst was removed by filtration and the filtrate evaporated to dryness. The solid was dissolved in the minimum of ether and to this solution was added a solution of HCl gas in ether. The green solution was cooled to -18°C and the precipitated solid isolated by filtration. This solid was dissolved in hot ethanol, treated with charcoal, filtered hot, and ether added to the filtrate. The solid isolated was dried _in vacuo_ at 55° and also by azeotropic removal of water with toluene. The solid was again dried _in vacuo_ affording the title product mp 168-9°C.

Analysis

Found: C 62.06; H 8.34; N 4.94; Cl 12.27%

Theory for $C_{15}H_{23}NO_2.HCl.\frac{1}{4}H_2O$

C 62.06; H 8.44; N 4.8; Cl 12.24%

Example 7

7-(Aminomethyl)-4-hydroxybicyclo[4.2.0]octa-1,3,5-triene-3-methanol

(a) 3-Formyl-4-methoxybicyclo[4.2.0]octa-1,3,5-triene-7-carbonitrile

4-Methoxybicyclo[4.2.0]octa-1,3,5-triene-7-carbonitrile (3.85g, 0.024 moles) and titanium tetrachloride (9.1g, 0.048 moles) in dichloromethane (70 mole) were cooled to $-5^{o}$ and 1,1-dichlorodimethyl ether (2.76g, 0.024 moles) added dropwise. The mixture was kept at $20^{o}$ for 1 hour, then poured into ice-water and stirred for 30 minutes. The organic layer was evaporated to dryness and the residue partitioned between diethyl ether and 40% sodium bisulphite solution (100ml). The bisulphite layer was basified with aqueous sodium hydroxide solution and extracted with ethyl acetate. Evaporation of the ethyl acetate extracts gave the sub-title compound.

(b) 3-Formyl-4-hydroxybicyclo[4.2.0]octa-1,3,5-triene-7-carbonitrile

The product of step (a) (0.94g, 5 mmoles) in dichloromethane (20ml) was cooled to $-80^{o}$ and boron trichloride (1.8g, 15 mmoles) was added under nitrogen. The mixture was allowed to warm to $20^{o}$ and stirred under nitrogen for 24 hours. Methanol (5ml) was added, the solution evaporated to dryness and the residue chromatographed on

silica using toluene:ethyl acetate mixtures as eluent. The centre fraction eluted was evaporated to dryness giving the sub-title compound as pale yellow crystals (0.25g, 29%) mp 131-134°.

(c) 7-(Aminomethyl)-4-hydroxybicyclo[4.2.0]octa-1,3,5-triene-3-methanol hydrochloride

The product of step (b) (0.105g, 0.6 mmoles) in dry tetrahydrofuran (15ml) containing 1M borane-tetrahydrofuran complex (2ml, 2 mmoles) was boiled under reflux under nitrogen for 4 hours. Methanol (5ml) and methanolic hydrogen chloride solution (5ml) were added. The solution was stirred for 1 hour then evaporated to yield the sub-title compound as a white solid m.p. 270° (charring) (114mg, 87%) NMR (DMSO-$d_6$) $\delta$ 2.8-3.5 (m,5H,CH's), 4.66(S,2H,$CH_2OH$), 6.87(S,1H,$H_5$), 7.04(S,1H,$H_2$), 8.4(bs,4H,OH+$NH_3^+$), 9.9(bs,1H,phenol OH).

<u>What we claim is</u>:-

1. A compound formula I,

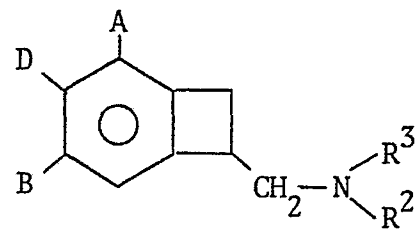

I

in which one of A, B and D represents hydrogen and the other two independently represent $-CH_2OR$ or -OR,

R represents hydrogen or phenylalkyl, and

$R^2$ and $R^3$, which may be the same or different, each represent hydrogen, alkyl or unsubstituted or substituted phenylalkyl,

and the pharmaceutically acceptable salts thereof.

2. A compound according to Claim 1, wherein A or B is hydrogen and R is hydrogen.

3. A compound according to Claim 1 or Claim 2, wherein $R^2$ and $R^3$ are hydrogen or alkyl C 1 to 6.

4. A compound according to Claim 3, wherein $R^2$ and $R^3$ are both alkyl C 1 to 6.

5. A compound according to Claim 1, wherein when R, $R^2$ or $R^3$ represents phenylalkyl the alkyl moiety of which is C 1 to 4.

6. 2,3-Dihydroxybicyclo[4.2.0]octa-1,3,5-triene-7-(N,N-di-n-propyl)methanamine.

7. 3,4-Dihydroxybicyclo[4.2.0]octa-1,3,5-triene-7-(N,N-di-n-propyl)methanamine.

8. 3,4-Dihydrocybicyclo[4.2.0]octa-1,3,5-triene-7-methanamine,

3,4-Dihydroxybicyclo[4.2.0]octa-1,3,5-triene-7-N-[2-(3,4-dimethoxyphenyl)ethyl]methanamine,

3,4-dibenzyloxybicyclo[4.2.0]octa-1,3,5-triene-7-N-[2-(3,4-dimethoxyphenyl)ethyl]methanamine,

3,4-dihydroxybicyclo[4.2.0]octa-1,3,5-triene-7-N-(1-methylethyl]methanamine,

3,4-dibenzyloxybicyclo[4.2.0]octa-1,3,5-triene-7-N-[1-methylethyl]methanamine,

2,3-dihydroxybicyclo[4.2.0]octa-1,3,5-triene-7-(N,N-dimethyl)methanamine,

2,3-dibenzyloxybicyclo[4.2.0]octa-1,3,5-triene-7-(N,N-di-n-propyl)methanamine,

3,4-dibenzyloxybicyclo[4.2.0]octa-1,3,5-triene-7-(N,N-di-n-propyl)methanamine,

7-(aminomethyl)-4-hydroxybicyclo[4.2.0]octa-1,3,5-triene-3-methanol,

or a pharmaceutically acceptable salt of any one thereof.

9. A pharmaceutical composition comprising a compound according to any one of the preceding claims in admixture with a pharmaceutically acceptable adjuvant, diluent or carrier.

10. A process for the production of a compound of formula I,

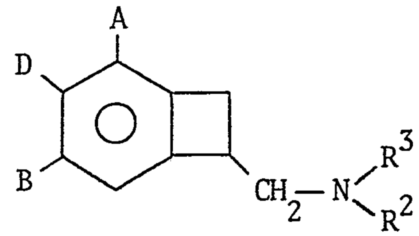

I

in which one of A, B and D represents hydrogen and the other two independently represent $-CH_2OR$ or $-OR$,

R represents hydrogen or phenylalkyl, and

$R^2$ and $R^3$, which may be the same or different, each represent hydrogen, alkyl or unsubstituted or substituted phenylalkyl,

or a pharmaceutically acceptable salt thereof,

wherein a compound of formula II,

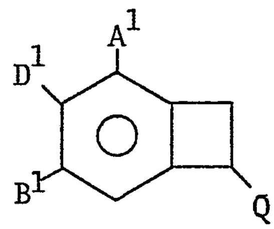

II

in which one of $A^1$, $B^1$ and $D^1$ represents hydrogen and the other two independently represent $-CHO$, $-CH_2OR^1$ or $-OR^1$,

$R^1$ represents a blocking group or a group R as defined above, and

Q represents $-CH_2NR^2R^3$, $-CN$ or $-CONR^2R^3$ where $R^2$ and $R^3$ are as defined above, provided that $R^1$ does not represent hydrogen when Q represents $-CH_2NR^2R^3$,

is reduced or hydrolysed to give the corresponding

compound of formula I,

and where desired or necessary converting the resulting compound of formula I to a pharmaceutically acceptable salt thereof or vice versa.

11. A compound of formula II as defined in Claim 10.

CLAIMS FOR AUSTRIA

What we claim is:-

1. A process for the production of a compound of formula I,

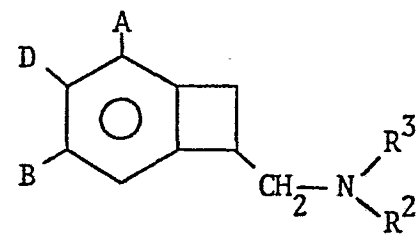

I

in which one of A, B and D represents hydrogen and the other two independently represent $-CH_2OR$ or $-OR$, R represents hydrogen or phenylalkyl, and $R^2$ and $R^3$, which may be the same or different, each represent hydrogen, alkyl or unsubstituted or substituted phenylalkyl,

or a pharmaceutically acceptable salt thereof, wherein a compound of formula II,

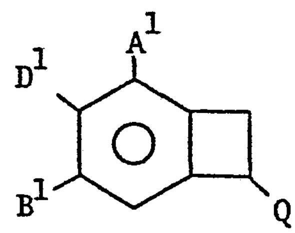

II

in which one of $A^1$, $B^1$ and $D^1$ represents hydrogen and the other two independently represent $-CHO$, $-CH_2OR^1$ or $-OR^1$,

$R^1$ represents a blocking group or a group R as defined above, and

Q represents $-CH_2NR^2R^3$, $-CN$ or $-CONR^2R^3$ where $R^2$ and $R^3$ are as defined above, provided that $R^1$ does not repre-

sent hydrogen when Q represents $-CH_2NR^2R^3$,

is reduced or hydrolysed to give the corresponding compound of formula I,

and where desired or necessary converting the resulting compound of formula I to a pharmaceutically acceptable salt thereof or vice versa.

2. A process according to Claim 1, wherein A or B is hydrogen and R is hydrogen.

3. A process according to Claim 1 or Claim 2, wherein $R^2$ and $R^3$ are hydrogen or alkyl C 1 to 6.

4. A process according to Claim 1, wherein $R^2$ and $R^3$ are both alkyl C 1 to 6.

5. A process according to Claim 1, wherein when R, $R^2$ or $R^3$ represents phenylalkyl the alkyl moiety of which is C 1 to 4.

6. A process according to Claim 1, wherein the compound of formula I is 2,3-dihydroxybicyclo[4.2.0]octa-1,3,5-triene-7-(N,N-di-n-propyl)methanamine or a pharmaceutically acceptable salt thereof.

7. A process according to Claim 1, wherein the compound of formula I is 3,4-dihydroxybicyclo[4.2.0]octa-1,3,5-triene-7-(N,N-di-n-propyl)methanamine or a pharmaceutically acceptable salt thereof.

8. A process according to Claim 1, wherein the compound of formula I is 3,4-dihydroxybicyclo[4.2.0]octa-1,3,5-

triene-7-methanamine,

3,4-dihydroxybicyclo[4.2.0]octa-1,3,5-triene-7-N-[2-(3,4-dimethoxyphenyl)ethyl]methanamine,

3,4-dibenzyloxybicyclo[4.2.0]octa-1,3,5-triene-7-N-[2-(3,4-dimethoxyphenyl)ethyl]methanamine,

3,4-dihydroxybicyclo[4.2.0]octa-1,3,5-triene-7-N-(1-methylethyl]methanamine,

3,4-dibenzyloxybicyclo[4.2.0]octa-1,3,5-triene-7-N-[1-methylethyl]methanamine,

2,3-dihydroxybicyclo[4.2.0]octa-1,3,5-triene-7-(N,N-dimethyl)methanamine,

2,3-dibenzyloxybicyclo[4.2.0]octa-1,3,5-triene-7-(N,N-di-n-propyl)methanamine,

3,4-dibenzyloxybicyclo[4.2.0]octa-1,3,5-triene-7-(N,N-d-n-propyl)methanamine,

7-(aminomethyl)-4-hydroxybicyclo[4.2.0]octa-1,3,5-triene-3-methanol,

or a pharmaceutically acceptable salt of any one thereof.